(19) Europäisches Patentamt
European Patent Office
Office européen des brevets

(11) EP 0 575 304 B1

(12) # EUROPEAN PATENT SPECIFICATION

(45) Date of publication and mention of the grant of the patent: **25.09.1996 Bulletin 1996/39**

(51) Int Cl.[6]: **C07C 409/40**, C07C 409/42, C11D 3/39

(21) Application number: **93870110.9**

(22) Date of filing: **14.06.1993**

(54) **Sulfonamide peroxycarboxylic acids**

Sulfonamido Peroxycarbonsäure

Acides sulfonamido peroxycarboxyliques

(84) Designated Contracting States:
**AT BE CH DE DK ES FR GB IE IT LI NL PT SE**

(30) Priority: **15.06.1992 US 898665**

(43) Date of publication of application:
**22.12.1993 Bulletin 1993/51**

(73) Proprietor: **Akzo Nobel N.V.**
**NL-6824 BM Arnhem (NL)**

(72) Inventors:
- **Chou, Yueting**
**Chesterfield, Missouri 63017 (US)**
- **Morris, Donald Eugene**
**Kirkwood, Missouri 63122 (US)**

(74) Representative: **Schalkwijk, Pieter Cornelis et al**
**AKZO NOBEL N.V.**
**Patent Department (Dept. APTA)**
**P.O. Box 9300**
**6800 SB Arnhem (NL)**

(56) References cited:
**EP-A- 0 260 134**
**EP-A- 0 290 292**
**EP-A- 0 334 427**
**EP-A- 0 399 584**
**EP-A- 0 484 324**
**EP-A- 0 485 927**
**US-A- 3 183 266**

- **CHEMICAL ABSTRACTS, vol. 76, no. 21, 22 May 1972, Columbus, Ohio, US; abstract no. 126066p, page 422 ;column 1 ;**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 575 304 B1

Printed by Jouve, 75001 PARIS (FR)

## Description

This invention relates to dry, stable bleaches comprising a sulfonamide peroxycarboxylic acid having a sulfonamide group and attached thereto organic moieties containing at least one peroxycarboxylic acid group.

## BACKGROUND OF THE INVENTION

The present invention relates to dry, stable bleaching compositions comprising a sulfonamide peroxycarboxylic acid compound having surprising inherent properties providing active oxygen bleaching performance even after long storage periods.

The property possessed by some materials to bleach is known and widely used to remove discoloration or stains from articles. The behavior and mechanisms by which such bleaching agents perform their functions are only partially understood. It is known that many colored materials contain a conjugated chain, that is, a series of double bonds which alternate with single bonds. If one of the double bonds is eliminated the color is usually destroyed. Therefore, an agent which will remove a double bond linkage may be an effective bleach. A bleaching agent may also act on the groups at the end of the chain. Bleaching materials are generally categorized as chlorine, hypochlorites, chloramines, hydrogen peroxide and other peroxy compounds, chlorite and chlorine dioxide as well as reducing agents.

One well known category of bleaches comprises active chlorine releasing compounds. Bleaches in this category, while effective, have the disadvantages of tending to weaken or degrade fabrics or other materials, to react with other components of formulations containing them, to degrade the colors of many dyed fabrics or other colored articles and to cause yellowing of some synthetic or resin treated fabrics, etc. The disadvantages of the active chlorine releasing bleaches are largely overcome by a second known category of bleaches referred to as inorganic oxygen bleaches comprising inorganic active oxygen releasing compounds. Bleaches in this category, while effective, have also exhibited significant disadvantages. For example, inorganic oxygen bleaches such as hydrogen peroxide, sodium perborate, sodium percarbonate, and the like, while being thermally and hydrolytically stable, suffer the serious disadvantage that they must be used at a relatively high temperature such as 85°C. or higher to be optimally effective in the absence of costly activators. A trend toward lower washing temperatures renders them unacceptable for use in many household washing machines which are now being operated at water temperatures less than 60°C. In general, effectiveness at lower temperatures would be advantageous because of reduced energy costs, reduced fabric damage or shrinkage, reduced need for sorting out temperature sensitive articles, etc.

To overcome the unsatisfactory low temperature performance of inorganic oxygen releasing compounds, it has been proposed that they be used in combination with so called bleach activators. Generally, these bleach activators are compounds which react with an inorganic oxygen bleach during the bleaching operation to release, in situ, a more reactive oxygen bleach such as a peroxycarboxylic acid. Several serious disadvantages are involved in the use of such combinations of inorganic oxygen bleaches with bleach activators. For example, in typical practice it is necessary to employ a large excess of either the inorganic oxygen releasing compound or the activator in order to obtain an acceptably complete and rapid release of the effective bleaching species. Another disadvantage is that the bleach activator must contain within its structure moieties which, upon release of the effective bleaching species, become side products. These side products contribute little or nothing to bleaching. Thus, the inclusion of these moieties tends to be wasteful.

All of the above-mentioned disadvantages of chlorine bleaches and inorganic oxygen bleaches used alone or in combination with activators can be overcome by the use of effective organic oxygen bleaches, particularly by the use of peroxycarboxylic acids. A number of such peroxycarboxylic acid bleaches are known in the art. However, these prior art peroxycarboxylic acids also exhibit some significant disadvantages. For example, due to their relatively high reactivity, these compounds tend to be difficult to maintain during storage of products containing them, prior to their use. In some cases, it is impossible to achieve an acceptable shelf life. In other cases, it is necessary to use expensive stabilization systems which may consume large amounts of stabilizing materials. For example, if prior art peroxycarboxylic acid bleaches are incorporated into a complete detergent formulation, stabilization is possible only at substantial extra cost as by encapsulation or other means of ingredient segregation. Examples of prior art teaching the coating technique to isolate peroxycarboxylic acids are US-A-3 847 830 to Williams et al, US-A-4 094 808 to Stewart et al and US-A-4 321 301 to Brichard et al. Chemical Abstracts, 76, n°. 126066 describes unsymmetrical phenylene containing peroxycarboxylic acids. EP-A-0334427 discloses alkylsulfonyl peroxycarboxylic acids, methods of making them and their use in e.g. bleaching and detergent compositions. EP-A- 0485927 describes sulfimidoperoxycarboxylic acids and the use thereof in bleaching, oxidating, and desinfecting agents.

Other consequences of inherent molecular instability of peroxycarboxylic acids include the need to blend them with components capable of absorbing energy during their decomposition in order to prevent violent decomposition. See for example US-A-4 100 095 to Hutchins et al. A further disadvantage of some prior art peroxycarboxylic acids is a lack of selectivity in their bleaching action. Thus, in such cases, dyes on some colored articles are significantly damaged during bleaching, although usually not to as great an extent as with chlorine bleaches.

Several types of peroxycarboxylic acid bleaches are disclosed which attempt to overcome the above disadvantages. U-S-A-4 634 551 and US-A-4 686 063 disclose an amide substituted peroxycarboxylic acid bleach compositions. US-A-4 681 592 discloses a bleach composition having a peroxycarboxylic acid with a hereroatom-containing moiety in the carbon chain. US-A-4 758 369 an US-A-4 824 591 disclose sulfone peroxycarboxylic acid bleach compositions.

Numerous sulfonamides are known and are useful for various purposes. For example, US-A-5 103 054 discloses tertiary sulfonamides useful as antioxidants. Aryl sulfonamide herbicides are disclosed in JP-A-63-060979. Certain sulfonic acid amides have been found to be useful as anti-arrhythmic agents as disclosed in US-A-4 794 196, Bleach activators having the described in US-A-4 772 290 having a generic formula as follows:

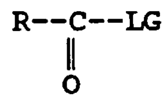

wherein "LG" is a leaving group which is displaced when the peracid forms and R is an organic residue of 1 to 20 carbon atoms. The compounds of the above formula may also contain an additional group attached to either the R or LG portion of the molecule, one such group being a sulfonamide of the formula

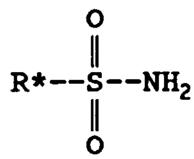

wherein R* is an organic linking group typically having less than about 8 carbon atoms. Carboxamides and sulfonamides functioning as latent acid catalysts for polymerization of cationically-sensitive monomers is disclosed in US-A-4 332 954. Preferred sulfonamides disclosed therein are represented by the formula

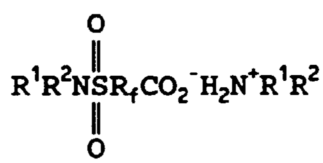

wherein $R_f$ is a perfluoroalkylene having 2 to 5 backbone or catenary carbon atoms or perfluorocycloalkylene having 4 to 7 ring atoms $R_1$ and $R^2$ are independently hydrogen, or a monovalent organic radical containing from 1 to 20 carbon atoms.

Bleaching compounds and composition comprising fatty peroxyacids salts thereof and precursors therefor having amide moieties in the fatty chain are disclosed in US-A-4 634 551. Amide peracids and bleach activators are disclosed.

None of the references shown above disclose the present invention of a sulfonamide peroxycarboxylic acid bleach composition.

## SUMMARY OF THE INVENTION

There has now been discovered a new class of peroxycarboxylic acids generally described as sulfonamide peroxycarboxylic acids. Some advantages of many sulfonamide peroxycarboxylic acids include means of their preparation which are unusually efficient, employment of low cost raw materials in their production, and physical properties which are favorable for efficiently incorporating them in various formulated products.

Sulfonamide peroxycarboxylic acids in accordance with this invention are represented by the following formula:

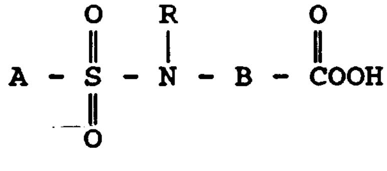

and

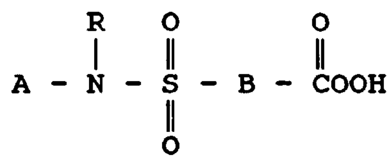

wherein A and B are peroxycarboxylic acid compatible organic moieties, bonded to the sulfur or nitrogen atoms by a non-carbonyl carbon atom and R is selected from the group consisting of hydrogen and $C_{1-3}$ alkyl. When B represents an alkylene group it is preferred that the group is free of alkyl substitution, that is B is a straight chain alkylene group.

As employed herein "peroxycarboxylic acid compatible" means that the moiety or any substituent group thereon does not react with the peroxycarboxylic acid group under normal conditions of storage and use of the claimed peracids.

## DETAILED DESCRIPTION OF THE INVENTION

Any number of suitable organic moieties can be employed to provide the intermediate link between the peroxyacid group and the sulfonamide group. For example, organic moieties may be employed to modify the solubility of the compound at point of use. In fact, organic moieties A and B in the above formula may be the same or different. Variation of organic moieties A and B allows for tailoring desirable compounds through choice of the organic moiety to lend specific properties to the molecule. In the preferred embodiment the compounds of this invention possess at least some degree of water solubility. The solubility of the compounds of this invention is, of course, modified by pH conditions at point of use such as in detergent baths.

Preferably, organic moieties A and B of the above formula are selected from the group consisting of cyclic, linear or branched alkyl hydrocarbyl groups containing from 1 to 16 carbon atoms (more preferably from 2 to 10 carbon atoms), aryl groups, aromatic heterocyclic groups, polyaryl groups consisting of from 2 to about 4 annelated benzenoid rings, and combinations thereof. Also, organic moieties A and B can be substituted with essentially any peroxycarboxylic acid compatible group or groups selected from hydroxy, halogen (chloro, bromo, or fluoro), sulfonate, nitro, carboxylic acid, carboxylate salt or ester, phenyl, $C_1$-$C_4$ alkoxy (e.g. ethoxy), heteroaryl, sulfone, amine oxide, amide, ester, nitrile and sulfate groups and the like to replace a hydrogen atom attached to the organic moieties A or B. The organic moieties A and B may not contain substituents which would react readily with the active oxygen from the peroxyacid group. Common reactive groups may include iodides, ketones, aldehydes, sulfoxides, sulfides, mercaptans, amines, reactive olefins, etc.

The groups A and B may contain any number of combinations of aromatic rings, alkyl chains, substituted aromatic rings, and substituted alkyl chains provided only that all substituents are stable in the presence of a peracid group.

Preferred substituents are located to provide adequate stability and are selected from the group consisting of chloro, nitro, alkyl, aryl, ester,

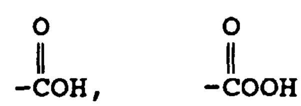

and amide.

A particularly preferred class of peroxyacids of this invention is represented by the above formula I wherein A and B are peroxycarboxylic acid compatible hydrocarbyl groups.

The acid is then oxidized by conventional means with a suitable oxidizing agent such as hydrogen peroxide.

Included among the hydrocarbyl moieties are alkyl, aralkyl inclusive of cyclic, straight and branched chain radicals, such as methyl, ethyl, isopropyl, cyclopropyl, cyclohexyl, tertiary butyl, n-butyl and the various forms of amyl, hexyl, heptyl, octyl, nonyl, decyl, undecyl, dodecyl, tridecyl, tetradecyl, pentadecyl, hexadecyl, heptadecyl, octadecyl, benzyl, phenylethyl, naphthylethyl, tolylethyl, methylbenzyl, phenylbenzyl and the like, aryl groups and alkaryl groups such as phenyl, biphenyl, tolyl, xylyl, naphthyl, and the like. It is preferred that A is alkyl of 1 to 8 carbon atoms and B is alkyl of from 2 to 4 carbon atoms. R is selected from the group consisting of hydrogen and alkyl radicals having from 1 to 3 carbon atoms. R is preferably hydrogen or methyl. R may also represent ethyl and propyl radicals including n-propyl and iso-propyl moieties.

The novel peroxycarboxylic acids of this invention are prepared from the corresponding carboxylic acids, esters, anhydrides, etc. in conventional manner. In a typical procedure the sulfonamide acid precursor is reacted with hydrogen peroxide in an acidic medium such as sulfuric acid or methanesulfonic acid. Isolation of the sulfonamide peroxycarboxylic acid is performed in the usual manner for recovering solids since most of the novel peroxycarboxylic acids of this invention are normally solid at room temperature.

The precursor acids can be prepared from the reaction of a sulfonyl chloride with an amino acid according to the

following schemes:

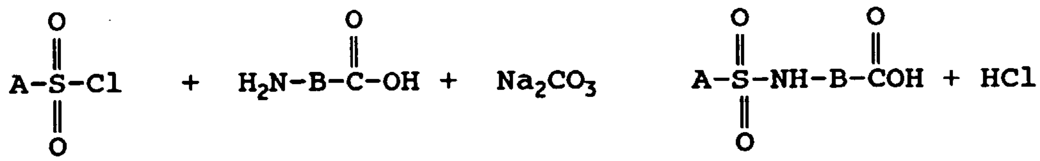

and

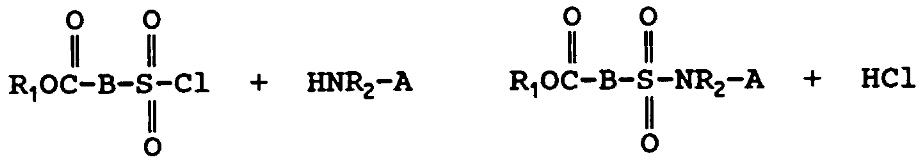

wherein A and B have the same meaning as in Formula I above. Typical amino acids include alpha amino acids such as alanine, arginine, asparagine, aspartic acid cysteine, glutamine, glutamic acid, glycine, histidine, isoleucine, leucine, lysine, methionine, phenylalanine, proline, serine, threonine, tryptophan, tyrosine and valine whether the L, D or DL form. Preferred amines and amino acids contain linear non-branched alkyl groups to which the amine group is bonded.

In the above formulas, it is preferred that A be an alkyl group having from six to fourteen carbon atoms while preferred B groups are alkylene groups having two or three carbon atoms. As noted above R is preferably hydrogen or a methyl group.

The acid is then oxidized by conventional means with a suitable oxidizing agent such as hydrogen peroxide.

It is recommended that sulfonamide carboxylic acid precursors which are not sufficiently soluble in the acidic medium during peroxidation be converted to the ester form using low molecular weight alkyl alcohols such as methyl, ethyl or propyl alcohols. The ester form is more often easily peroxidized to the desired sulfone peroxycarboxylic acid. It has been observed that the sulfone group is relatively stable and withstands vigorous peroxidation procedures.

Other acids useful in the peroxidation reaction include various sulfonic acids and strong acid cation exchange resins. Generally, peroxidation is conducted at temperatures in the range of from 0°C. to 75°C. depending upon the reactivity of the precursor and the stability of the precursor and the resulting peroxycarboxylic acid.

Generally, it is preferred to employ a stoichiometric excess of peroxidizing agent and then separate the excess agent after peroxidation. Any suitable peroxidizing agent may be employed. Hydrogen peroxide is preferred.

Mixtures of sulfonamide peroxycarboxylic acids with each other and/or with the corresponding carboxylic acids or esters are included within the scope of this invention. Such mixtures nearly always result when precursors containing two or more groups convertible to peroxycarboxylic acid groups (such as -COOH, -COOR where R is lower alkyl and the like) are reacted with hydrogen peroxide to produce a peroxycarboxylic acid composition. In such mixtures, it is preferred that a substantial fraction such as 50% or more of the resulting molecules have all such groups converted to the peroxy acid group in order to make efficient use of the precursor.

The water solubility of the sulfonamide peroxycarboxylic acids of this invention can be varied in one or more ways known to one skilled in the art. For example, inclusion of a long alkyl chain tends to depress the water solubility, especially as the number of carbon atoms increases. Also in many cases solubility tends to decrease as molecular weight increases. In any given series of compounds varying primarily in water solubility an optimum degree of water solubility will exist and this can be determined by routine experimentation. In most cases, a relatively low water solubility, i.e. less than about 1% by weight, is preferred because this facilitates efficient separation of the product from excess $H_2O_2$ and acid catalysts used during preparation.

Typical compounds of this invention are described below with respect to the following formula.

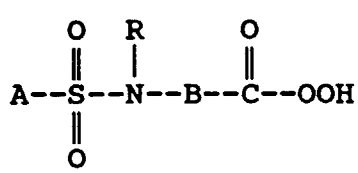

and

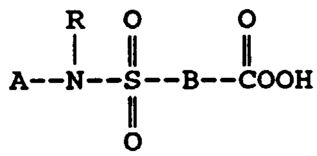

wherein A, R and B are as indicated in the table below

| A | R | B |
|---|---|---|
| octyl | hydrogen | ethylene |
| phenyl | hydrogen | ethylene |
| octyl | hydrogen | phenylene |
| phenyl | hydrogen | phenylene |
| nonyl | hydrogen | ethylene |
| butyl | methyl | ethylene |
| nonyl | hydrogen | phenylene |
| octyl | hydrogen | propylene |
| octyl | methyl | ethylene |
| nonyl | hydrogen | propylene |
| nonyl | methyl | ethylene |
| decyl | hydrogen | ethylene |
| decyl | hydrogen | propylene |
| decyl | hydrogen | phenylene |
| decyl | methyl | ethylene |
| phenyl | hydrogen | propylene |
| phenyl | methyl | ethylene |

Compounds of this invention can be employed in a variety of modes. Not only can they be employed in dry bleach formulations but also they can be employed in hard surface cleaners, laundry detergents, and machine dishwashing compositions as well as a wide variety of other compositions useful for laundry or other purposes.

The laundry detergent compositions of this invention comprise from 2 percent to 80 percent of a detergent surfactant, detergent builder or mixtures thereof and from .1 percent to 50 percent of the novel sulfonamide peroxycarboxylic acids of this invention.

Preferably the compositions contain from 5 percent to 30 percent detergent surfactant, from 0 percent to 50 percent detergent builder and from .5 percent to 20 percent of the sulfonamide peroxycarboxylic acids of this invention to give from .05 percent to 3 percent available oxygen.

Suitable detergent compositions and detergent ingredients are disclosed in US-A-4 166 039, US-A-4 157 978, US-A-4 056 481, US-A-4 049 586, US-A-4 035 257, US-A-4 019 998, US-A-4 000 080, and US-A-3 983 078 all of which are incorporated herein by reference. Disclosures of additional ingredients appear in US-A-4 089 945, US-A-3 987 161 and US-A-3 962 418 incorporated herein by reference. Preferably, the compositions are in solid granular or particulate form and preferably are formulated to prevent reaction of other ingredients with the active oxygen in the novel sulfonamide peroxycarboxylic acids of this invention.

The dry bleach compositions of this invention comprise from 0 percent to 50 percent detergent surfactant, detergent builder or mixtures thereof and from 1 percent to 50 percent of the stable sulfonamide peroxycarboxylic acids of this invention. Preferably the compositions contain from 5 percent to 30 percent detergent surfactant, from 0 percent to 50 percent detergent builder and from .5 percent to 25 percent of the sulfoneamide peroxycarboxylic acids of this invention to give .05 percent to 3 percent active oxygen.

In the following examples, which illustrate the invention, and throughout the specification, parts and percent are by weight unless otherwise indicated.

EXAMPLE 1

(Preparation of (N-propionic acid) octylsulfonamide-precursor)

Into a 250cc four-necked flask equipped with a stirrer and a thermometer was placed 10.6g (0.10 mole) sodium carbonate and 60cc water. While the mixture was warmed, 3.62g (0.04 mole) of β-alanine was added, and the temperature was raised to 60°C. Then 10g o(1.047 mole) octylsulfonyl chloride was added gradually in 30 minutes at 60-63°C. After the addition, the mixture was held at 60-70°C for an additional 90 minutes. The temperature was raised to about 85°C and one spoonful of activated charcoal sold under the trade name Norit by American Norit Company was added, and the solution was filtered by suction through a previously heated funnel. The filtrate was cooled and slowly added with 6N HCl to a pH of 2. The product was isolated by filtration. About 4.4g of crude product was obtained. Some product was lost in the filtrate because of its relatively high solubility in water. The product was recrystallized from toluene and gave a melting point of 118°C. The elemental analyses supported the structure for (N-propionic acid) octylsulfonamide as follows:

| Element | Found % | Theoretical % |
|---|---|---|
| C | 49.75 | 49.81 |
| H | 8.76 | 8.68 |
| S | 5.36 | 5.28 |
| N | 12.02 | 12.07 |

EXAMPLE 2

(Preparation of (N-propionic peracid) Octylsulfonamide)

To a 50cc peroxidation apparatus equipped with a stirrer and a thermometer was placed 1g (3.77 m mole) of (N-propionic peracid) octylsulfonamide and 0.8cc of concentrated sulfuric acid. The mixture was stirred and warmed to 28°C. Over a period of about 20 minutes, 1g (26.5 m mole) of 90% hydrogen peroxide was added drop-wise. The temperature of the mixture was maintained at about 33°C during the addition. After completion of the addition of hydrogen peroxide the mixture was cooled and ice added. At a temperature of 2°C the mixture was filtered and the cake was washed with ice water, then dried under vacuum and air dried. The solid product provided 4.77% available oxygen which is about 84% of the theoretical value and exhibited a melting point in the range of 79°-80°C. The crude peracid was mixed with a an equal weight of boric acid for stability during the testing period.

EXAMPLE 3

BLEACHING PERFORMANCE

In all of the tests below a detergent is employed as a control at a use level of 1.5 g/L of wash solution. The peracid of Example 2 was added to portions of the detergent composition in the amount needed to provide 4 ppm available oxygen. Each test series contained a control. The detergent formulation is as follows:

| Ingredient | Weight % |
|---|---|
| Sodium alkyl benzene sulfonate | 16 |
| Sodium carbonate | 10 |
| Sodium silicate (47% solids) | 9 |
| Water | 8 |
| Carboxymethyl cellulose | 1 |
| Sodium sulfate | 24 |
| Sodium tripolyphosphate | 32 |

The wash conditions of 100°F and water having a hardness level of 150 ppm (3:2 mole ratio of calcium to magnesium calculated as calcium carbonate) was used. In each test a set of three swatches were evenly stained. After staining, the light reflectance value (Rdi) was measured using the Gardner XL-23 Tristimulator Colorimeter manufactured by Gardner Laboratory, Inc., Bethesda, Md.

A Terg-o-tometer was employed to test the bleaching performance of the bleach compounds. in each test three stained swatches together with three unstained swatches were placed in a cylindrical container with 1 liter of water and 1.4g of detergent together with a weighed amount of a bleach compound of this invention. Two minutes were allowed for the detergent to dissolve. The washing operation covered a period of 10 minutes after which the laundered swatches were rinsed with clear water and dried. Light reflectance measurements of each cleaned dried swatch were made and averaged ($Rd_f$). the difference ($\Delta Rd$) of these readings for each type of stain was determined ($\Delta Rd = Rd_f - Rd_i$).

The $\Delta R_d$ data is employed to indicate the percent stain removal (%SR) which may be related to visual effect. Percent stain removal is reported below calculated according to the formula:

$$\frac{\Delta R_d}{100 - Rd_i} \times 100 = \text{percent stain removal}$$

In the table below there is listed the various tests comparing the bleaching ability of the product of Example 2 (X below) with octyl sulfonyl perbutyric acid (Y below). Clay and tea stains on cotton cloth were employed to determine bleach performance. The amount of X and Y in the table below is stated in grams. The compositions tested were as follows:

Table I

| Run No. | X | Y | Stain | pH |
|---|---|---|---|---|
| 1 | - | - | Clay | 10.3 |
| 2 | .1878 | - | Clay | 8.85 |
| 3 | - | .1527 | Clay | 8.85 |
| 4 | - | - | Tea | 10.3 |
| 5 | .1878 | - | Tea | 8.85 |
| 6 | - | .1527 | Tea | 8.85 |

Table II

| Percent Stain Removal | | | |
|---|---|---|---|
| Run No. | Control | X | Y |
| 1 | 47 | | |
| 2 | | 60 | |
| 3 | | | 52 |
| 4 | 2 | | |
| 5 | | 25 | |
| 6 | | | 20 |

Although the invention has been described in terms of specific embodiments which are set forth inconsiderable detail, it would be understood that this description is by way of illustration only and that the invention is not necessarily limited thereto since alternative embodiments and operating techniques will become apparent to those skilled in the art in view of this disclosure. Accordingly, modifications are contemplated which can be made without departing from the spirit of the described invention.

## Claims

1. A compound represented by one of the following formulas:

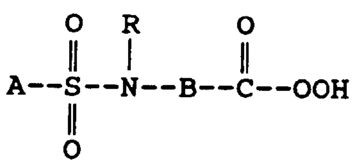

and

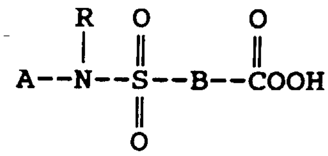

wherein A and B are alkyl hydrocarbyl groups containing 1 to 16 carbon atoms which can be substituted by one or more groups selected from hydroxy, halogen, sulfonate, nitro, carboxylic acid, carboxylate salt DA ester, phenyl, $C_1$ - $C_4$ alkoxy, heteroaryl, sulfone, amine oxide, amide, ester, nitrile, and sulfate groups and R is selected from the group consisting of hydrogen and alkyl radicals having from 1 to 3 carbon atoms.

**2.** A compound of Claim 1 wherein R is hydrogen.

**3.** A compound of Claim 1 wherein B is ethylene.

**4.** A compound of Claim 1 wherein A is octyl, R is hydrogen and B is ethylene

**5.** A compound of Claim 1 wherein R is alkyl.

**6.** A compound of Claim 5 wherein the alkyl is methyl.

**7.** A compound of Claim 1 wherein A is alkyl having from 6 to 14 carbon atoms, B is an alkylene group having 2 or 3 carbon atoms and R is selected from the group consisting of hydrogen and a methyl group.

**8.** A bleaching detergent composition comprising a detergent surfactant and an effective amount of a compound represented by one of the formulas:

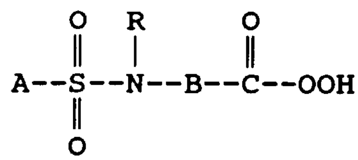

and

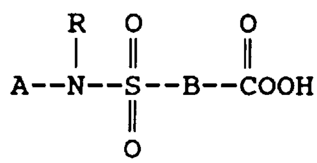

wherein A and B are alkyl hydrocarbyl groups containing 1 to 16 carbon atoms which can be substituted by one or more groups selected from hydroxy, halogen, sulfonate, nitro, carboxylic acid, carboxylate salt DA ester, phenyl, $C_1$ - $C_4$ alkoxy, heteroaryl, sulfone, amine oxide, amide, ester, nitrile, and sulfate groups and R is selected from the group consisting of hydrogen and alkyl radicals having from 1 to 3 carbon atoms.

**9.** A composition of Claim 8 wherein R is hydrogen.

**10.** A composition of Claim 8 wherein B is ethylene.

**11.** A composition of Claim 8 wherein A is octyl, R is hydrogen and B is ethylene.

**12.** A composition of Claim 8 wherein R is alkyl.

**13.** A composition of Claim 12 wherein the alkyl is methyl.

**14.** A bleaching detergent composition of Claim 8 wherein A is alkyl having from 6 to 14 carbon atoms, B is an alkylene group having 2 or 3 carbon atoms and R is selected from the group consisting of hydrogen and a methyl group.

15. A dry bleach composition comprising, by weight, from 0 percent to 50 percent detergent surfactant, detergent builder or a mixture thereof and from 1 percent to 50 percent of a compound represented by one of the following formulas:

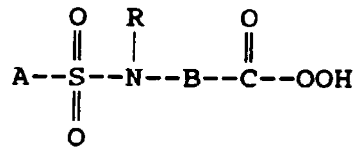

and

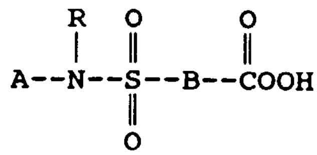

wherein A and B are alkyl hydrocarbyl groups containing 1 to 16 carbon atoms which can be substituted by one or more groups selected from hydroxy, halogen, sulfonate, nitro, carboxylic acid, carboxylate salt DA ester, phenyl, $C_1$ - $C_4$ alkoxy, heteroaryl, sulfone, amine oxide, amide, ester, nitrile, and sulfate groups and R is selected from the group consisting of hydrogen and alkyl radicals having from 1 to 3 carbon atoms.

16. A composition of Claim 15 wherein R is hydrogen.

17. A composition of Claim 15 wherein A and B are hydrocarbyl groups.

18. A composition of Claim 15 wherein B is ethylene.

19. A composition of Claim 15 wherein A is octyl, R is hydrogen and B is ethylene

20. A composition of Claim 15 wherein R is alkyl.

21. A composition of Claim 20 wherein the alkyl is methyl.

22. a process for bleaching articles comprising contacting the article with a compound represented by one of the formulas:

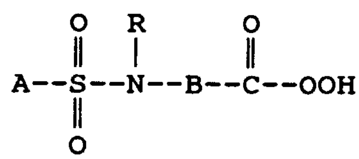

and

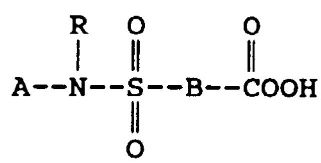

wherein A and B are alkyl hydrocarbyl groups containing 1 to 16 carbon atoms which can be substituted by one or more groups selected from hydroxy, halogen, sulfonate, nitro, carboxylic acid, carboxylate salt DA ester, phenyl, $C_1$ - $C_4$ alkoxy, heteroaryl, sulfone, amine oxide, amide, ester, nitrile, and sulfate groups and R is selected from the group consisting of hydrogen and alkyl radicals having from 1 to 3 carbon atoms.

23. A process of Claim 22 wherein R is hydrogen.

24. A process of Claim 23 wherein A and B are hydrocarbyl groups.

25. A process of Claim 22 wherein B is ethylene.

**26.** A process of Claim 22 wherein A is octyl, R is hydrogen and B is ethylene

**27.** A process of Claim 22 wherein R is alkyl.

**28.** A process of Claim 27 wherein the alkyl is methyl.

**29.** A dry bleach composition of Claim 15 wherein A is alkyl having from 6 to 14 carbon atoms, B is an alkylene group having 2 or 3 carbon atoms and R is selected from the group consisting of hydrogen and a methyl group.

**30.** A process of Claim 22 wherein A is alkyl having from 6 to 14 carbon atoms, B is an alkylene group having 2 or 3 carbon atoms and R is selected from the group consisting of hydrogen and a methyl group.

## Patentansprüche

**1.** Verbindung einer der folgenden Formeln:

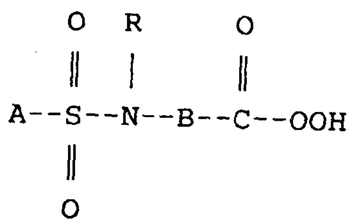

und

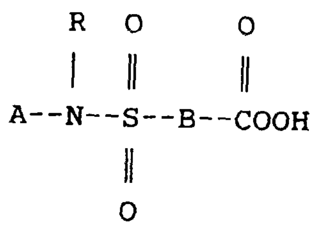

worin A und B Alkylhydrocarbyl-Gruppen mit 1 bis 16 Kohlenstoffatomen bedeuten, die substituiert sein können durch eine oder mehrere Gruppen, ausgewählt aus Hydroxy-, Halogen-, Sulfonat-, Nitro-, Carbonsäure-, Carboxylatsalz- oder -ester-, Phenyl-, $C_1$-$C_4$-Alkoxy-, Heteroaryl-, Sulfon-, Aminoxid-, Amid-, Ester-, Nitril- und Sulfat-Gruppen; und R ausgewählt ist aus der Gruppe bestehend aus Wasserstoff und Alkylresten mit 1 bis 3 Kohlenstoffatomen.

**2.** Verbindung nach Anspruch 1, worin R Wasserstoff darstellt.

**3.** Verbindung nach Anspruch 1, worin B Ethylen ist.

**4.** Verbindung nach Anspruch 1, worin A Octyl bedeutet, R Wasserstoff darstellt, und B Ethylen ist.

**5.** Verbindung nach Anspruch 1, worin R Alkyl bedeutet.

**6.** Verbindung nach Anspruch 5, worin das Alkyl Methyl darstellt.

**7.** Verbindung nach Anspruch 1, worin A Alkyl mit 6 bis 14 Kohlenstoffatomen bedeutet, B eine Alkylen-Gruppe mit 2 oder 3 Kohlenstoffatomen darstellt, und R ausgewählt ist aus der Gruppe bestehend aus Wasserstoff und einer Methyl-Gruppe.

**8.** Bleichdetergens-Zusammensetzung, welche umfaßt: ein Detergens-Surfactant und eine wirksame Menge einer Verbindung einer der Formeln:

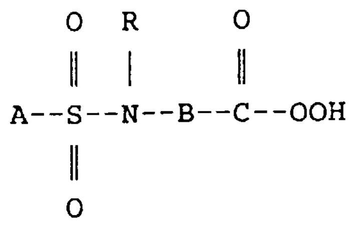

und

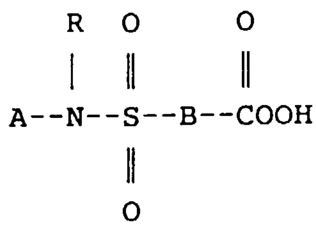

worin A und B Alkylhydrocarbyl-Gruppen mit 1 bis 16 Kohlenstoffatomen bedeuten, die substituiert sein können durch eine oder mehrere Gruppen, ausgewählt aus Hydroxy-, Halogen-, Sulfonat-, Nitro-, Carbonsäure-, Carboxylatsalz- oder -ester-, Phenyl-, $C_1$-$C_4$-Alkoxy-, Heteroaryl-, Sulfon-, Aminoxid-, Amid-, Ester-, Nitril- und Sulfat-Gruppen; und R ausgewählt ist aus der Gruppe bestehend aus Wasserstoff und Alkylresten mit 1 bis 3 Kohlenstoffatomen.

**9.** Zusammensetzung nach Anspruch 8, worin R Wasserstoff darstellt.

**10.** Zusammensetzung nach Anspruch 8, worin B Ethylen ist.

**11.** Zusammensetzung nach Anspruch 8, worin A Octyl bedeutet, R Wasserstoff darstellt, und B Ethylen ist.

**12.** Zusammensetzung nach Anspruch 8, worin R Alkyl bedeutet.

**13.** Zusammensetzung nach Anspruch 12, worin das Alkyl Methyl darstellt.

**14.** Bleichdetergens-Zusammensetzung nach Anspruch 8, worin A Alkyl mit 6 bis 14 Kohlenstoffatomen bedeutet, B eine Alkylen-Gruppe mit 2 oder 3 Kohlenstoffatomen darstellt, und R ausgewählt ist aus der Gruppe bestehend aus Wasserstoff und einer Methyl-Gruppe.

**15.** Trockenbleichzusammensetzung, welche, bezogen auf die Masse, umfaßt: 0 bis 50 % Detergens-Surfactant, Detergens-Builder oder eine Mischung hievon und 1 bis 50 % einer Verbindung einer der folgenden Formeln:

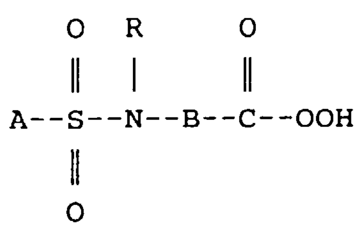

und

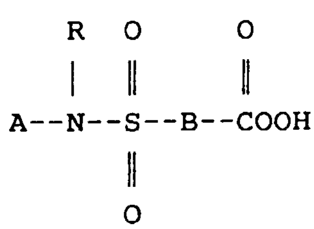

worin A und B Alkylhydrocarbyl-Gruppen mit 1 bis 16 Kohlenstoffatomen bedeuten, die substituiert sein können

durch eine oder mehrere Gruppen, ausgewählt aus Hydroxy-, Halogen-, Sulfonat-, Nitro-, Carbonsäure-, Carboxylatsalz- oder -ester-, Phenyl-, $C_1$-$C_4$-Alkoxy-, Heteroaryl-, Sulfon-, Aminoxid-, Amid-, Ester-, Nitril- und Sulfat-Gruppen; und R ausgewählt ist aus der Gruppe bestehend aus Wasserstoff und Alkylresten mit 1 bis 3 Kohlenstoffatomen.

**16.** Zusammensetzung nach Anspruch 15, worin R Wasserstoff darstellt.

**17.** Zusammensetzung nach Anspruch 15, worin A und B Hydrocarbyl-Gruppen bedeuten.

**18.** Zusammensetzung nach Anspruch 15, worin B Ethylen ist.

**19.** Zusammensetzung nach Anspruch 15, worin A Octyl bedeutet, R Wasserstoff darstellt, und B Ethylen ist.

**20.** Zusammensetzung nach Anspruch 15, worin R Alkyl bedeutet.

**21.** Zusammensetzung nach Anspruch 20, worin das Alkyl Methyl darstellt.

**22.** Verfahren zum Bleichen von Artikeln, welches umfaßt: Inberührungbringen des Artikels mit einer Verbindung einer der Formeln:

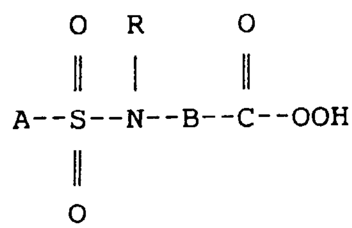

und

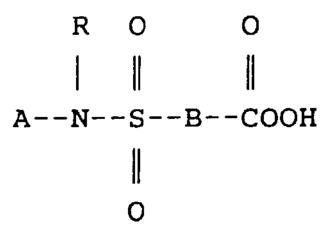

worin A und B Alkylhydrocarbyl-Gruppen mit 1 bis 16 Kohlenstoffatomen bedeuten, die substituiert sein können durch eine oder mehrere Gruppen, ausgewählt aus Hydroxy-, Halogen-, Sulfonat-, Nitro-, Carbonsäure-, Carboxylatsalz- oder -ester-, Phenyl-, $C_1$-$C_4$-Alkoxy-, Heteroaryl-, Sulfon-, Aminoxid-, Amid-, Ester-, Nitril- und Sulfat-Gruppen; und R ausgewählt ist aus der Gruppe bestehend aus Wasserstoff und Alkylresten mit 1 bis 3 Kohlenstoffatomen.

**23.** Verfahren nach Anspruch 22, wobei R Wasserstoff darstellt.

**24.** Verfahren nach Anspruch 23, wobei A und B Hydrocarbyl-Gruppen bedeuten.

**25.** Verfahren nach Anspruch 22, wobei B Ethylen ist.

**26.** Verfahren nach Anspruch 22, wobei A Octyl bedeutet, R Wasserstoff darstellt, und B Ethylen ist.

**27.** Verfahren nach Anspruch 22, wobei R Alkyl bedeutet.

**28.** Verfahren nach Anspruch 27, wobei das Alkyl Methyl darstellt.

**29.** Trockenbleichzusammensetzung nach Anspruch 15, worin A Alkyl mit 6 bis 14 Kohlenstoffatomen bedeutet, B eine Alkylen-Gruppe mit 2 oder 3 Kohlenstoffatomen darstellt, und R ausgewählt ist aus der Gruppe bestehend aus Wasserstoff und einer Methyl-Gruppe.

30. Verfahren nach Anspruch 22, wobei A Alkyl mit 6 bis 14 Kohlenstoffatomen bedeutet, B eine Alkylen-Gruppe mit 2 oder 3 Kohlenstoffatomen darstellt, und R ausgewählt ist aus der Gruppe bestehend aus Wasserstoff und einer Methyl-Gruppe.

**Revendications**

1. Composé représenté par l'une des formules suivantes :

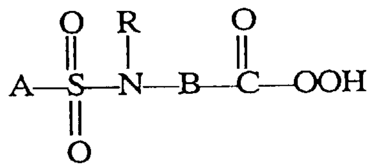

et

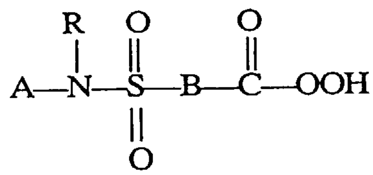

dans laquelle A et B représentent des groupes hydrocarbonés de type alkyle comportant de 1 à 16 atomes de carbone qui peuvent être substitués par un ou plusieurs groupes choisis parmi les groupes hydroxy, sulfonate, nitro, carboxyle, carboxyle sous forme de sel ou d'ester, phényle, alcoxy en $C_1$-$C_4$, hétéro-aryle, oxyde d'amine, amide, ester, nitrile, sulfate, un atome d'halogène, et R est choisi dans l'ensemble formé par un atome d'hydrogène, des radicaux alkyle comportant de 1 à 3 atomes de carbone.

2. Composé conforme à la revendication 1, dans lequel R représente un atome d'hydrogène.

3. Composé conforme à la revendication 1, dans lequel B représente un groupe éthylène.

4. Composé conforme à la revendication 1, dans lequel A représente un groupe octyle, R représente un atome d'hydrogène, et B représente un groupe éthylène.

5. Composé conforme à la revendication 1, dans lequel R représente un groupe alkyle.

6. Composé conforme à la revendication 5, dans lequel le groupe alkyle est le groupe méthyle.

7. Composé conforme à la revendication 1, dans lequel A représente un groupe alkyle comportant de 6 à 14 atomes de carbone, B représente un groupe alkylène comportant de 2 à 3 atomes de carbone et R est choisi dans l'ensemble constitué par un atome d'hydrogène et un groupe méthyle.

8. Composition détergente de blanchiment comprenant un tensio-actif détergent et une quantité efficace d'un composé représenté par l'une des formules

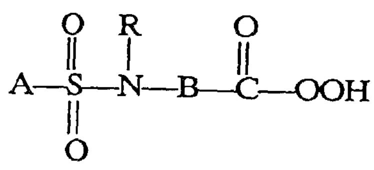

et

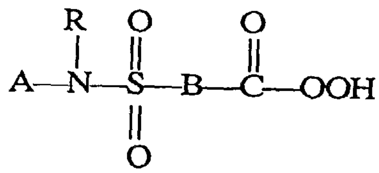

dans lesquelles A et B représentent des groupes hydrocarbonés de type alkyle comportant de 1 à 16 atomes de carbone qui peuvent être substitués par un ou plusieurs groupes choisis parmi les groupes hydroxy, sulfonate, nitro, carboxyle, carboxyle sous forme de sel ou d'ester, phényle, alcoxy en $C_1$-$C_4$, hétéro-aryle, oxyde d'amine, amide, ester, nitrile, sulfate, un atome d'halogène, et R est choisi dans l'ensemble formé par un atome d'hydrogène, des radicaux alkyle comportant de 1 à 3 atomes de carbone.

**9.** Composition conforme à la revendication 8, dans laquelle R représente un atome d'hydrogène.

**10.** Composition conforme à la revendication 8, dans laquelle B représente un groupe éthylène.

**11.** Composition conforme à la revendication 8, dans laquelle A représente un groupe octyle, R représente un atome d'hydrogène, et B représente un groupe éthylène.

**12.** Composition conforme à la revendication 8, dans laquelle R représente un groupe alkyle.

**13.** Composition conforme à la revendication 12, dans laquelle le groupe alkyle est le groupe méthyle.

**14.** Composition détergente de blanchiment conforme à la revendication 8, dans laquelle A représente un groupe alkyle comportant de 6 à 14 atomes de carbone, B représente un groupe alkylène comportant de 2 à 3 atomes de carbone et R est choisi dans l'ensemble constitué par un atome d'hydrogène et un groupe méthyle.

**15.** Composition de blanchiment sèche comprenant en poids, de 0 % à 50 % de tensio-actif détergent, d'un adjuvant actif de détergence ou d'un mélange de ces derniers, et de 1 % à 50 % d'un composé correspondant à l'une des formules suivantes

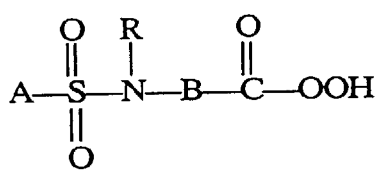

et

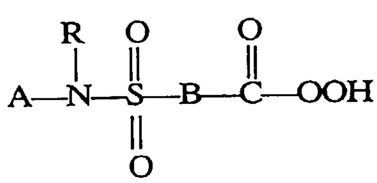

dans lesquelles A et B représentent des groupes hydrocarbonés de type alkyle comportant de 1 à 16 atomes de carbone qui peuvent être substitués par un ou plusieurs groupes choisis parmi les groupes hydroxy, sulfonate, nitro, carboxyle, carboxyle sous forme de sel ou d'ester, phényle, alcoxy en $C_1$-$C_4$, hétéro-aryle, oxyde d'amine, amide, ester, nitrile, sulfate, un atome d'halogène, et R est choisi dans l'ensemble formé par un atome d'hydrogène, des radicaux alkyle comportant de 1 à 3 atomes de carbone.

**16.** Composition conforme à la revendication 15, dans laquelle R représente un atome d'hydrogène.

**17.** Composition conforme à la revendication 15, dans laquelle A et B sont des groupes hydrocarbonés.

**18.** Composition conforme à la revendication 15, dans laquelle B représente un groupe éthylène.

**19.** Composition conforme à la revendication 15, dans laquelle A représente un groupe octyle, R représente un atome d'hydrogène, et B représente un groupe éthylène.

**20.** Composition conforme à la revendication 15, dans laquelle R représente un groupe alkyle.

**21.** Composition conforme à la revendication 20, dans laquelle le groupe alkyle est le groupe méthyle.

**22.** Procédé qui consiste à blanchir des articles et qui comprend le fait de mettre l'article en contact avec un composé représenté par l'une des formules

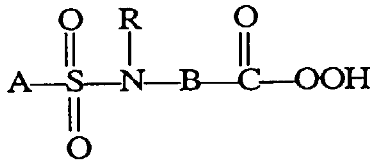

et

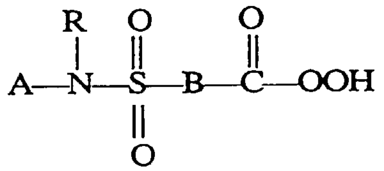

dans lesquelles A et B représentent des groupes hydrocarbonés de type alkyle comportant de 1 à 16 atomes de carbone qui peuvent être substitués par un ou plusieurs groupes choisis parmi les groupes hydroxy, sulfonate, nitro, carboxyle, carboxyle sous forme de sel ou d'ester, phényle, alcoxy en $C_1$-$C_4$, hétéro-aryle, oxyde d'amine, amide, ester, nitrile, sulfate, un atome d'halogène, et R est choisi dans l'ensemble formé par un atome d'hydrogène, des radicaux alkyle comportant de 1 à 3 atomes de carbone.

**23.** Procédé conforme à la revendication 22, dans lequel R représente un atome d'hydrogène.

**24.** Procédé conforme à la revendication 23, dans lequel A et B sont des groupes hydrocarbonés.

**25.** Procédé conforme à la revendication 22, dans lequel B représente un groupe éthylène.

**26.** Procédé conforme à la revendication 22, dans lequel A représente un groupe octyle, R représente un atome d'hydrogène, et B représente un groupe éthylène.

**27.** Procédé conforme à la revendication 22, dans lequel R représente un groupe alkyle.

**28.** Procédé conforme à la revendication 27, dans lequel le groupe alkyle est le groupe méthyle.

**29.** Composition de blanchiment sèche conforme à la revendication 15, dans laquelle A représente un groupe alkyle comportant de 6 à 14 atomes de carbone, B représente un groupe alkylène comportant 2 ou 3 atomes de carbone, et R est choisi dans l'ensemble constitué par un atome d'hydrogène et un groupe méthyle.

**30.** Procédé conforme à la revendication 22, dans lequel A représente un groupe alkyle comportant de 6 à 14 atomes de carbone, B représente un groupe alkylène comportant 2 ou 3 atomes de carbone, et R est choisi dans l'ensemble constitué par un atome d'hydrogène et un groupe méthyle.